# EUROPÄISCHE PATENTSCHRIFT

(11) **EP 1 959 184 B1**
(45) Veröffentlichungstag und Bekanntmachung des Hinweises auf die Patenterteilung: **16.01.2013**
(21) Anmeldenummer: 08002229.6
(22) Anmeldetag: 07.02.2008
(51) Int. Cl.: F16M 11/06, A61B 19/00

(54) **Haltevorrichtung für medizinische Zwecke**
Holder for medicinal purposes
Dispositif de retenue à des fins médicales

(30) Priorität: 13.02.2007 DE 102007006892
(43) Veröffentlichungstag der Anmeldung: 20.08.2008
(73) Patentinhaber: University of Dundee, Dundee DD1 4HN (GB)
(72) Erfinder: Brown, Stuart I., Dr., Fife KY16 8QX (GB); Rutherford, Ian, Dundee DD4 0RL (GB)
(74) Vertreter: Hofmeister, Frank

(56) Entgegenhaltungen:
- EP-A2- 0 730 320
- WO-A1-00/43685
- WO-A1-2004/053382
- DE-A1- 3 026 160
- US-A- 5 824 007

## Beschreibung

Die Erfindung betrifft eine Haltevorrichtung für medizinische Zwecke mit einem Tragarm, an dessen distalem Ende mindestens ein medizinisches Instrument in einer Instrumentenaufnahme festlegbar ist und mit mindestens einem Gelenk zur Positionierung des Tragarms und/oder des medizinischen Instruments, wobei das mindestens eine Gelenk zwischen einer das Gelenk freigebenden und einer das Gelenk arretierenden Stellung verstellbar ist, wobei das mindestens eine Gelenk aus mindestens zwei Gelenkteilen besteht, die über ineinandergreifende Rastelemente in Wirkverbindung miteinander treten, die im Wesentlichen senkrecht zur Längsrichtung des Tragarms ausgerichtet sind, und wobei im Gelenk mindestens ein hydraulisch oder pneumatisch aufweitbares Expansionselement angeordnet ist, über das das mindestens eine Gelenk hydraulisch oder pneumatisch in die das Gelenk freigebende Stellung überfühbar ist.

Derartige Haltevorrichtungen sind bei der Durchführung chirurgischer Eingriffe häufig erforderlich, um medizinische Instrumente verschiedenster Art, wie beispielsweise Retraktoren, Kameras oder Endoskope, über einen längeren Zeitraum in einer bestimmten Position zu halten. Durch die gelenkige Ausgestaltung der Haltevorrichtungen ist es für den Chirurgen möglich, das in der Instrumentenaufnahme gehaltene medizinische Instrument exakt zu positionieren und die eingestellte Position der Haltevorrichtung durch Arretieren des Gelenks bzw. der Gelenke zu fixieren.

Eine gattungsgemäße Haltevorrichtung ist beispielsweise aus der WO 2004/053382 A1 bekannt. Bei dieser bekannten Vorrichtung besteht ein jedes Gelenk aus zwei Gelenksegmenten, die über eine ineinandergreifende Verzahnung in Wirkverbindung miteinander treten. Weiterhin ist in jedem Gelenk ein pneumatisch betätigbarer Kolben angeordnet, über den das Gelenk in eine das Gelenk freigebende Stellung überführbar ist. Die gegenseitige Verzahnung ist dabei an einem Gelenksegment sowie am Kolben ausgebildet. Darüber hinaus sind die Gelenksegmente über eine Federelement in die das Gelenk freigebende Stellung vorgespannt. Die Anordnung eines pneumatisch betätigbaren Kolbens in jedem Gelenk bedeutet einen sehr hohen fertigungstechnischen Aufwand.

Aus der WO 00/43685 A1 ist weiterhin eine einstellbare Positionierungsvorrichtung bekannt, die einen aus mehreren Teilen bestehenden Tragarm aufweist, wobei die einzelnen Tragarmteile über festellbare und wieder lösbare Gelenke miteinander verbunden sind. Jedes Gelenk ist mit einer luftdruckbetriebenen Kupplung versehen, um das Lösen und wieder Festlegen der Gelenke zu bewirken. Die Anordnung einer luftdruckbetriebenen Kupplung in jedem Gelenk bedeutet ebenfalls einen sehr hohen fertigungstechnischen Aufwand.

Eine weitere Haltevorrichtung ist beispielsweise aus der US 2003/116167 A1 bekannt. Bei dieser bekannten Haltevorrichtung bestehen die Gelenke aus zwei Gelenkteile, die über ineinandergreifende Rastelemente in Wirkverbindung miteinander treten, wobei jedes Gelenk eine aufblasbare Blase aufweist, die im expandierten Zustand die Rastelemente in Eingriff miteinander drückt. Nachteilig an dieser bekannten Konstruktion ist, dass die Tragarmteile bei einem Druckabfall in der Blase sofort relativ zueinander verschwenken, weil der gegenseitige Eingriff der Rastelemente der Gelenkteile ausschließlich über den aufgebrachten Druck der Blase erzeugt wird.

Eine weitere Haltevorrichtung ist beispielsweise aus der DE 195 26 915 B4 bekannt. Bei dieser bekannten Haltevorrichtung werden die Gelenkteile reibschlüssig durch die Federkraft mindestens eines Federelements relativ zueinender arretiert. Das Lösen der Arretierung erfolgt pneumatisch über im Gelenk angeordnete Lamellen, die beispielsweise mit Druckluft beaufschlagt für eine Trennung des Reibschlusses sorgen. Zwar haben sich derartige Haltevorrichtungen in der Praxis durchaus bewährt, jedoch bilden gerade die über den Reibschluss zusammenzuhaltenden Kontaktflächen Probleme bei der Reinigung, da aufgrund der hohen Kontaktkräfte, die notwendig sind, um einen zuverlässigen Reibschluss zu erzeugen, Kratzer in den Kontaktflächen erzeugt werden können, die ihrerseits Keimzellen für Verunreinigungen bilden können.

Weiterhin ist eine Haltevorrichtung aus der DE 43 16 590 A1 bekannt. Bei dieser bekannten Haltevorrichtung ist die Gelenkmechanik als Zahnradgetriebe ausgebildet, wobei die Verzahnung des als Innen- und Außenzahnkranz ausgebildeten Zahnradgetriebes parallel zur Längsrichtung des Tragarms, also parallel zur Drehachse ausgerichtet ist und die Zahnkränze über eine axial wirkende Druckkraft in Eingriff miteinander gehalten werden. Nachteilig an dieser Konstruktion ist, dass es einer hochpräzisen Fertigung der Zahnkränze bedarf, um ein spielfreies Ineinandergreifen der Zähne der beiden Zahnkränze zu gewährleisten. Die axiale Federkraft verhindert nur das Trennen der beiden Zahnkränze, die Verhinderung des Bewegungsspiels in horizontaler Richtung ist mit dieser Ausgestaltung nicht möglich.

Davon ausgehend liegt der Erfindung die Aufgabe zugrunde, eine Haltevorrichtung für medizinische Zwecke zu schaffen, die bei einfacher Handhabung und zuverlässiger Positionierbarkeit gut zu reinigen ist.

Die Lösung dieser Aufgabenstellung ist erfindungsgemäß dadurch gekennzeichnet, dass die ineinandergreifende Rastelemente an den Gelenkteilen angeordnet sind und die Gelenkteile über mindestens ein Federelement gegeneinander vorgespannt sind und, dass das im Gelenk angeordnete Expansionselement als Schlauch ausgebildet ist.

Die Ausbildung des Expansionselements als durch das Gelenk geführter Schlauch stellt eine besonders einfache und kostengünstige Art dar, um das pneumatische oder hydraulische Trennen der Gelenkteile bewerkstelligen zu können. Zur Lagerung des Schlauchs im Gelenk ist in mindestens einem der miteinander in Wirkverbindung bringbaren Gelenkteile eine Durchgangsöffnung zum Durchführen des Schlauches ausgebildet.

Aufgrund der axialen Vorspannung der Gelenkteile und dem Einsatz des aufweitbaren Expansionselements ausschließlich zum Freigeben der Gelenkverbindung, verbleibt die erfindungsgemäße Haltevorrichtung auch bei einem Druckabfall in der positionsgenauen Lage.

Durch die Ausbildung der Gelenkmechanik als im Wesentlichen senkrecht zur Längsrichtung des Tragarms ausgerichtete, ineinandergreifende Rastelemente ist es möglich eine Gelenkmechanik bereitzustellen, die einerseits einfach zu handhaben ist und andererseits eine im Wesentlichen spielfreie Positionierung des Tragarms gewährleistet. Durch das Ineinandergreifen der Rastelemente senkrecht zur Längsrichtung des Tragarms und die axial Vorspannung der Gelenkteile wird der Eingriff der Rastelemente ineinander verstärkt, ohne dass ein Bewegungsspiel für eine Relativbewegung der Gelenkteile zueinander verbleibt.

Um eine möglichst exakte Positionierung des auszurichtenden medizinischen Instruments zu ermöglichen, ist der Tragarm erfindungsgemäß vorteilhafterweise mehrteilig ausgebildet ist, wobei die einzelnen Tragarmteile über die Gelenke verschwenkbar miteinander verbunden sind.

Zur Ausbildung der Rastelemente wird mit einer bevorzugten Ausführungsform der Erfindung vorgeschlagen, dass die Rastelemente als an den einander zugewandten Stirnflächen der Gelenkteile ausgebildete Verzahnungen ausgebildet sind. Die Ausbildung der Rastelemente in der Art einer Hirth-Verzahnung ermöglicht auf einfache Art und Weise ein exaktes abgestuftes Einstellen der gewünschten Halteposition des Tragarms. Durch die Verwendung von zwei oder mehr Gelenken an einer Haltevorrichtung lassen sich auch bei der Verwendung von Zahnradgetrieben als Gelenkmechanik beliebige Einstellungswinkel erzeugen. Mit jedem zusätzlichen Gelenk wird der Bewegungsspielraum des Tragarms um einen Freiheitsgrad erhöht.

Mit einer bevorzugten Ausführungsform der Erfindung wird vorgeschlagen, dass zumindest ein Gelenkteil mehrteilig so ausgebildet ist, dass diese Teilelemente koaxiale Zahnkranzringe aufweisen, wobei die Zahnkranzringe der Teilelemente vorteilhafterweise in der das Gelenk freigebenden Stellung unabhängig voneinander relativ zueinander verdrehbar sind und in der das Gelenk arretierenden Stellung verdrehfest gegeneinander blockiert sind.

Die Kopplung der koaxialen Zahnkränze untereinander erfolgt erfindungsgemäß über eine Nut-Feder-Verbindung, die eine gegenseitige Führung der Zahnkränze gewährleistet.

Gemäß einer praktischen Ausführungsform der Erfindung wird vorgeschlagen, dass ein Gelenkteil zweiteilig aufgebaut ist und aus einem inneren zylindrischen Kern und einem den Kern koaxial umgebenden äußeren Ring besteht.

Um sicherzustellen, dass der gegenseitige Eingriff der Verzahnungen auch bei unterschiedlich großen Gewichtskräften an den Enden der über das Gelenk miteinander zu verbindenden Bauteile und den damit verbundenen unterschiedlichen Hebelkräften jederzeit gewährleistet ist, wird mit der Erfindung vorgeschlagen, dass beide über das Gelenk miteinander verbundenen Bauteile, insbesondere Tragarmteile, so an dem zweiteiligen Gelenkteil festgelegt sind, dass ein Bauteil/Tragarm mit dem inneren zylindrischen Kern verbunden ist und das andere BauteillTragarmteil mit dem äußeren Ring verbunden ist. Durch das Lagern beider BauteilelTragarmteile auf derselben Seite der Verzahnung wird vermieden, dass die auftretenden unterschiedlichen Kräfte die Verzahnung auseinander drücken.

Das gegenseitige Fixieren der Zahnkränze des mehrteilig aufgebauten Gelenkteils in der arretierten Stellung des Gelenks wird erfindungsgemäß vorteilhafterweise dadurch bewirkt, dass die Verzahnung des mit dem mehrteilig ausgebildeten Gelenkteil in Wirkverbindung stehenden anderen Gelenkteils so breit ausgebildet ist, dass diese in der das Gelenk arretierenden Stellung mit den allen Zahnkranzringen des mehrteiligen Gelenkteils in Eingriff steht.

Mit einer alternativen Ausführungsform der Erfindung wird vorgeschlagen, dass die Rastelemente als Stifte und Rastaufnahmen zur formschlüssigen Aufnahme der Stifte ausgebildet sind, wobei vorteilhafterweise die Rastaufnahmen als gelochte Ringscheibe an einem der Gelenkteile ausgebildet sind, die mit den korrespondierenden Stiften am anderen Gelenkteil in Eingriff bringbar ist.

Zur Erzeugung der gegenseitigen Vorspannung zwischen den beiden Gelenkteilen über das mindestens eine Federelement wird gemäß einer bevorzugten Ausführungsform der Erfindung vorgeschlagen, dass sich das mindestens eine Federelement zur Erzeugung der Vorspannung zwischen den Gelenkteilen an einem Gelenkteil abstützt und die Federkraft über ein mit dem Federelement verbundenes Spannelement auf das andere Gelenkteil überträgt, wobei das Spannelement vorteilhafterweise Rasthaken zum Festlegen am anzudrückenden Gelenkteil aufweist.

Die Vorspannung zwischen den beiden in Wirkverbindung stehenden Gelenkteilen ist erfindungsgemäß über das mindestens eine Federelement einstellbar, wobei diese Einstellung durch die Auswahl eines Federelements mit der gewünschten Federkennlinie oder durch Vorspannen des Federelements, beispielsweise über eine Spannschraube oder dergleichen, erfolgen kann.

Weiterhin wird mit der Erfindung vorgeschlagen, dass die Instrumentenaufnahme als Kugellager ausgebildet ist, wobei die Lagerkugel aus zwei Halbkugeln besteht und die Lagerschale aus zwei gegeneinander verspannbaren und mit Ausnehmungen für die Lagerkugel versehenen Platten besteht und wobei der Durchmesser der Ausnehmungen geringfügig kleiner als der Durchmesser der Lagerkugel.

Das eigentliche Halten des medizinischen Instruments in der Instrumentenaufnahme erfolgt erfindungsgemäß über eine im Bereich der Kontaktflächen der beiden die Lagerkugel bildenden Halbkugeln ausgebildete Durchgangsbohrung zur klemmenden Aufnahme des zu haltenden medizinischen Instruments.

Das Lösen der klemmenden Lagerung des medizinischen Instruments zwischen den beiden Halbkugeln des Kugellagers erfolgt erfindungsgemäß hydraulisch oder pneumatisch, wozu im Kugellager vorteilhafterweise mindestens ein hydraulisch oder pneumatisch aufweitbares Expansionselement angeordnet ist.

Schließlich wird einer praktischen Ausführungsform der Erfindung vorgeschlagen, dass das Expansionselement als Schlauch ausgebildet ist.

Weitere Merkmale und Vorteile der Erfindung ergeben sich anhand der zugehörigen Zeichnung, in der verschiedene Ausführungsbeispiele einer erfindungsgemä-βen Haltevorrichtung für medizinische Zwecke nur beispielhaft dargestellt sind, ohne die Erfindung auf diese Ausführungsbeispiele zu beschränken. In der Zeichnung zeigt:
- Fig. 1: eine Haltevorrichtung für medizinische Zwecke gemäß dem Stand der Technik;
- Fig. 2: eine schematische Seitenansicht einer ersten Ausführungsform eines Gelenks für eine erfindungsgemäße Haltevorrichtung für medizinische Zwecke;
- Fig. 3: eine schematische Seitenansicht einer zweiten Ausführungsform eines Gelenks für eine erfindungsgemäße Haltevorrichtung ;
- Fig. 4: eine perspektivische Ansicht des Gelenks gemäß Fig. 3;
- Fig. 5: eine schematische perspektivische Seitenansicht einer dritten Ausführungsform eines Gelenks für eine erfindungsgemäße Haltevorrichtung, das Gelenk in der arretierten Stellung darstellend;
- Fig. 6: eine Ansicht gemäß Fig. 5, jedoch das Gelenk in der freigegebenen Stellung darstellend und
- Fig. 7: eine schematische perspektivische Ansicht einer Instrumentenaufnahme für eine erfindungsgemäße Haltevorrichtung für medizinische Zwecke.

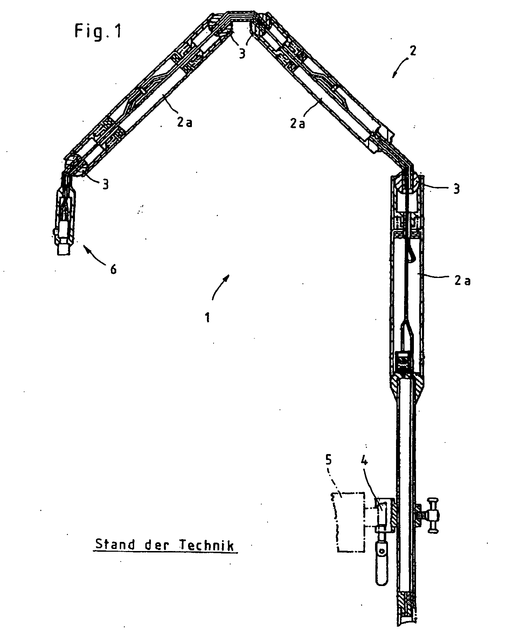

Die Abbildung Fig. 1 zeigt eine Haltevorrichtung 1 für medizinische Instrumente gemäß dem Stand der Technik. Diese Haltevorrichtung 1 besteht im Wesentlichen aus einem mehreren Tragarmteilen 2a bestehenden Tragarm 2, wobei die einzelnen Tragarmteile 2a des Tragarms über Gelenke 3 relativ zueinander verschwenkbar miteinander verbunden sind.

Derartige Haltevorrichtungen 1 sind bei der Durchführung chirurgischer Eingriffe häufig erforderlich, um medizinische Instrumente verschiedenster Art, wie beispielsweise Retraktoren, Kameras oder Endoskope, über einen längeren Zeitraum in einer bestimmten Position zu halten. Durch die gelenkige Ausgestaltung der Haltevorrichtung 1 ist es für den Chirurgen möglich, das medizinische Instrument exakt zu positionieren und die eingestellte Position der Haltevorrichtung 1 durch Arretieren des Gelenks 3 bzw. der Gelenke 3 zu fixieren. Neben der endoskopischen Chirurgie finden derartige Haltevorrichtungen 1 auch in der offenen Chirurgie Anwendung.

Im Bereich seines proximalen Endes ist der Tragarm 2 über eine Einspannvorrichtung 4, beispielsweise am Operationstisch 5, festlegbar. Am distalen Ende weist der Tragarm 2 eine Instrumentenaufnahme 6 zur Aufnahme des über die Haltevorrichtung 1 zu positionierenden medizinischen Instruments auf.

Alternativ zu dem in Fig. 1 dargestellten Aufbau des Tragarms 2 aus mehreren hintereinander gekoppelten Tragarmteilen 2a, die über Gelenke 3 miteinander verbunden sind, ist es selbstverständlich auch möglich den Tragarm 2 mehrarmig so auszugestalten, dass ausgehend von einem Gelenk 3 mehrere Tragarmteile 2a in verschiedene Richtungen zeigen. Diese Tragarmteile 2a ihrerseits können wieder über Gelenke 3 mit weiteren Tragarmteilen 2a gekoppelt sein und an ihren distalen Enden jeweils mit Instrumentenaufnahmen 6 zur Aufnahme der über die Haltevorrichtung 1 zu positionierenden medizinischen Instrumente ausgestattet sein.

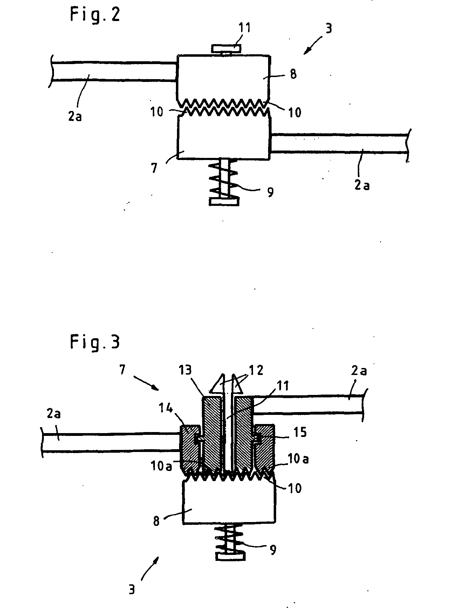

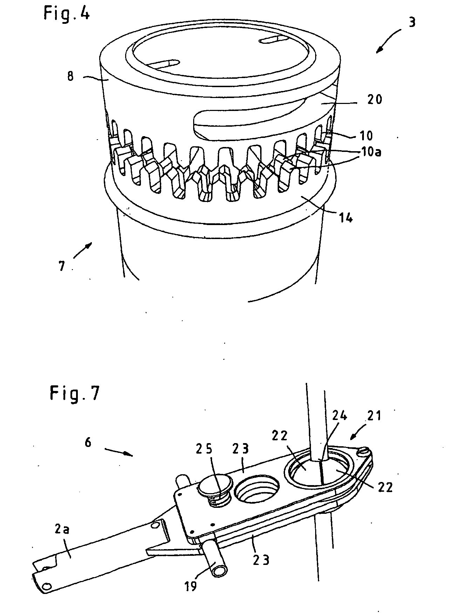

Der Aufbau der Gelenke 3 ist den Abbildungen Fig. 2 bis 6 und der Instrumentenaufnahme 6 der Abbildung Fig. 7 zu entnehmen.

Wie aus Fig. 2 bis 6 ersichtlich, bestehen die Gelenke 3 aus zwei Gelenkteilen 7 und 8, die über ineinandergreifende Rastelemente in Wirkverbindung miteinander treten, wobei die gegenseitigen Rastelemente im Wesentlichen senkrecht zur Längsrichtung des Tragarms 2 ausgerichtet sind und über mindestens ein Federelement 9 gegeneinander vorgespannt sind.

Bei den in den Abbildungen Fig. 2 bis 4 dargestellten beiden Ausführungsformen sind diese Rastelemente als an den einander zugewandten Stirnflächen der Gelenkteile 7 und 8 ausgebildete Verzahnungen 10 in der Art einer Hirth-Verzahnung ausgebildet. Die Zähne der Verzahnungen 10 sind dabei vorteilhafterweise sich konisch verjüngend ausgebildet, um ein automatisches und möglichst spielfreies Ineinandergleiten der Verzahnungen 10 zu erzielen, sobald die Gelenkteile 7 und 8 über das Federelement 9 gegeneinander gedrückt werden. Das direkte und totpunktfreie Ineinandergreifen der Verzahnungen 10 wird weiterhin dadurch erleichtert, dass die Spitzen der Zähne abgerundet oder abgeschrägt ausgebildet sind, so dass beim Aufeinandertreffen zweier Zähne der Verzahnungen 10 die Zähne immer seitlich in die die ineinandergreifende Verzahnung bewerkstelligende Position abgelenkt werden.

Wie weiterhin aus den Abbildungen Fig. 2 bis 4 ersichtlich, stützt sich das die Vorspannung zwischen den Gelenkteilen 7 und 8 des Gelenks 3 bewirkende Federelement 9 am Gelenkteil 7 oder 8 ab und überträgt die Federkraft über ein mit dem Federelement 9 verbundenes Spannelement 11 auf das andere Gelenkteil 8 oder 7.

Bei der in Fig. 3 dargestellten Ausführungsform weist das Spannelement 11 an seinem das Gelenkteil 7 überragenden freien Ende zusätzlich radial federelastische Rasthaken 12 zum Festlegen am anzudrückenden Gelenkteil 7 auf. Aufgrund der Federelastizität in radialer Richtung wirken die Rasthaken 12 wie ein Schnappverschluss, wodurch das auf das freie Ende des Spannelements 11 aufgesetzte Gelenkteil 7 ohne weiter Montagetätigkeit lagestabil in der richtigen Ausrichtung zum anderen Gelenkteil 8 gehalten wird. Zur Demontage des Gelenkteils 7 ist es bei dieser Ausgestaltungsform lediglich notwendig, die Rasthaken 12 radial nach innen zu drücken, um nachfolgend das Gelenkteil 7 vom Spannelement 11 abziehen zu können.

Um sicherzustellen, dass der gegenseitige Eingriff der Verzahnungen 10 auch bei unterschiedlich großen Gewichtskräften an den Enden der über das Gelenk 3 miteinander zu verbindenden Bauteile, beispielsweise der Tragarmteile 2a des Tragarms 2, und den damit verbundenen unterschiedlichen Hebelkräften jederzeit gewährleistet ist, sind bei der in den Abbildungen Fig. 3 und 4 dargestellten Ausführungsform beide über das Gelenk 3 miteinander verbundenen Bauteile/Tragarmteile 2a am selben Gelenkteil 7 festgelegt. Hierzu ist das Gelenkteil 7, wie insbesondere aus Fig. 3 ersichtlich, aus einem inneren zylindrischen Kern 13 und einem den Kern 13 koaxial umgebenden äußeren Ring 14 bestehend zweiteilig ausgebildet.

Durch das Lagern beider Bauteile/Tragarmteile 2a auf derselben Seite der Verzahnung 10 wird vermieden, dass die auftretenden unterschiedlichen Kräfte die Verzahnung 10 auseinander drücken.

Das zweiteilig Gelenkteil 7 ist so ausgebildet, dass die einzelnen Teilelemente, nämlich der Kern 13 und der Ring 14, koaxiale Zahnkranzringe 10a aufweisen, wobei die Zahnkranzringe 10a der Teilelemente 13, 14 in der das Gelenk 3 freigebenden Stellung unabhängig voneinander relativ zueinander verdrehbar sind und in der das Gelenk 3 arretierenden Stellung verdrehfest gegeneinander blockiert sind. Durch die mehrteilige Ausbildung eines Gelenkteils 7 mit den verdrehbaren Zahnkränzen 10a wird der Einstellungsspielraum der über das jeweilige Gelenk 3 miteinander zu verbindenden Bauteile zueinander deutlich erhöht.

Die Kopplung der beiden Teilelemente 13, 14 des Gelenkteils 7 untereinander und somit auch der koaxialen Zahnkränze 10a erfolgt über eine Nut-Feder-Verbindung 15, die eine gegenseitige Führung der Teilelemente 13, 14 bzw. der Zahnkränze 10a gewährleistet.

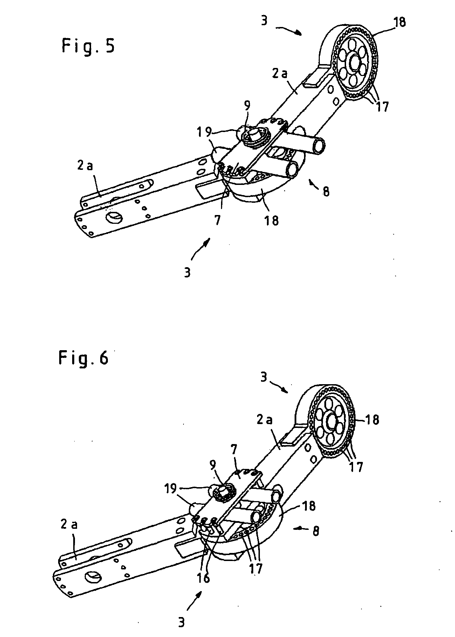

Bei der in den Abbildungen Fig. 5 und 6 dargestellten alternativen Ausführungsform sind die in Wirkverbindung miteinander tretenden und im Wesentlichen senkrecht zur Längsrichtung des Tragarms 2 ausgerichteten Rastelemente als Stifte 16 und Rastaufnahmen 17 zur formschlüssigen Aufnahme der Stifte 16 ausgebildet, wobei die Rastaufnahmen 17 als gelochte Ringscheibe 18 am Gelenkteil 8 ausgebildet sind, die über das Federelement 9 mit den am anderen Gelenkteil 7 angeordneten Stiften 16 in Eingriff bringbar ist.

Exemplarisch für alle zuvor beschriebenen Ausführungsformen zur Ausbildung der im Wesentlichen senkrecht zur Längsrichtung des Tragarms 2 ausgerichteten Rastelemente, nämlich der Verzahnungen 10 sowie der Kombination aus Stiften 16 und Rastaufnahmen 17 ist in den Abbildungen Fig. 5 und 6 dargestellt, wie das Gelenk 3 aus der das Gelenk 3 arretierenden Stellung, in der die Verzahnungen 10 kämmend ineinander greifen bzw. die Stifte 16 in die Rastaufnahmen 17 eingreifen, in eine das Gelenk 3 freigebende Stellung überführbar ist, in der das Gelenk 3 die positionsgenaue Einstellung der über das Gelenk 3 miteinander verbundenen Bauteile /Tragarmteile 2a relativ zueinander ermöglicht.

Das Überführen des Gelenks 3 in die das Gelenk 3 freigebende Stellung erfolgt hydraulisch oder pneumatisch über ein im Gelenk 3 angeordnetes Expansionselement 19, das bei der dargestellten Ausführungsform als Schlauch 19 ausgebildet ist. Alternativ Ausgestaltungsformen, wie beispielsweise als aufpumpbarer Luftbalg oder dergleichen, sind selbstverständlich auch einsetzbar.

Die Ausbildung des Expansionselements 19 als Schlauch 19 stellt eine besonders einfache und kostengünstige Variante dar. Auch aus hygienischer Sicht ist die Verwendung der als Schläuche 19 ausgebildeten Expansionselemente 19 vorteilhaft, da die Schläuche 19 einerseits gut sterilisierbar sind und andererseits aufgrund ihres geringen Preises auch als nicht zu reinigende Einwegartikel einsetzbar sind.

Bei der in Fig. 5 und 6 dargestellten Ausführungsform kommen zwei Schläuche 19 zum Einsatz, jedoch ist je nach konstruktiver Ausgestaltung des zu trennenden Gelenks 3 auch die Verwendung nur eines Schlauchs 19 ausreichend, wie dies beispielsweise der Abbildung Fig. 4 zu entnehmen ist, bei der im Gelenkteil 8 eine Durchgangsöffnung 20 zum Durchführen eines Schlauchs 19 ausgebildet ist.

In der in Fig. 5 dargestellten arretierten Stellung des Gelenks 3 werden die im Gelenk 3 zwischen den über das Federelement 9 ineinandergreifenden Gelenkteilen 7 und 8 angeordneten Schläuche 19 zusammengedrückt, bis die Gelenkteile 7 und 8 formschlüssig ineinander greifen.

Zum Lösen dieser formschlüssigen Verbindung werden die Schläuche 19 mit einem Fluid, beispielsweise Druckluft, beaufschlagt, wodurch die Schläuche 19 expandieren und die in Fig. 6 dargestellte Form einnehmen. Die Expansion der Schläuche 19 bewirkt, dass die Gelenkteile 7 und 8 außer Eingriff miteinander gedrückt werden, so dass das Gelenk 3 nachfolgend zur Ausrichtung des Tragarms 2 sowie des zu positionierenden medizinischen Instruments in eine neue Winkelstellung überführt werden kann.

Die Möglichkeit der hydraulischen oder pneumatischen Trennung der Gelenkverbindung hat in der Praxis enorme Vorteile, da das Freigeben des Gelenks 3 oder der Gelenke 3 ohne Zuhilfenahme eines Werkzeugs oder sonstige direkte manuelle Tätigkeit am Gelenk 3 erfolgen kann. Der Operateur muss lediglich über einen Hand- oder Fußschalter die Fluidzufuhr zu den Schläuchen 19 freigeben, um nachfolgend das betreffende Gelenk 3 neu positionieren zu können. Hierbei ist es selbstverständlich möglich, die einzelnen Gelenke 3 einer Haltevorrichtung 1 so mit der Fluidzufuhr zu verbinden, dass jedes Gelenk 3 einzeln ansteuerbar ist, einzelne Gelenke 3 zu Gelenkgruppen zusammengefasst sind oder alle Gelenke 3 gleichzeitig mit dem Fluid beaufschlagbar sind, um die Gelenke 3 in die freigebende Stellung zu überführen.

Darüber hinaus lässt sich die vom Fluid aufzubringende Druckkraft zum Trennen der formschlüssigen Verbindung der Gelenkteile 7 und 8 durch die über das Federelement 9 auf die Gelenkteile 7 und 8 ausgeübte Vorspannung durch die Auswahl entsprechender Druckfedern und/oder Vorspannen des Federelements 9, beispielsweise über eine Spannschraube, einstellen. So ist es auch bei der Reihenschaltung mehrerer Gelenke 3 an einer Fluidleitung möglich, die Freigabe der Gelenke 3 selektiv zu gestalten, wenn aufgrund der unterschiedlichen Vorspannungen der Gelenke 3 über die Federelemente 9 unterschiedliche Fluiddrücke erforderlich sind, um die Gelenkteile 7 und außer Eingriff miteinander zu bringen.

Fig. 7 zeigt schließlich eine mögliche Ausgestaltungsform zur Ausbildung der Instrumentenaufnahme 6.

Bei der dargestellten Ausführungsform ist die Instrumentenaufnahme 6 als Kugellager 21 ausgebildet ist, wobei die Lagerkugel aus zwei Halbkugeln 22 besteht und die Lagerschale aus zwei gegeneinander verspannbaren und mit Ausnehmungen für die Lagerkugel versehenen Platten 23 besteht und wobei der Durchmesser der Ausnehmungen geringfügig kleiner als der Durchmesser der Lagerkugel.

Zur klemmenden Aufnahme des zu haltenden medizinischen Instruments ist im Bereich der Kontaktflächen der beiden die Lagerkugel bildenden Halbkugeln 22 eine Durchgangsbohrung 24 ausgebildet, in der das medizinische Instrument Aufnahme findet. Sobald die beiden Platten 23 über das Federelement 25 zusammengedrückt werden, werden auch die in den Ausnehmungen der Platten 23 gelagerten Halbkugeln 22 so gegeneinander gepresst, dass das in der Durchgangsbohrung 24 angeordnete medizinische Instrument klemmend im Kugellager 21 gehalten wird.

Um die Montage und gegenseitige Lagerung der beiden Halbkugeln 22 zueinander zu erleichtern, können beispielsweise in Längsrichtung der Durchgangsbohrung 24 Führungselemente in der Art einer Nut-Feder-Verbindung angeformt werden.

Das Lösen der klemmenden Halterung im Kugellager 21 erfolgt auch hier hydraulisch oder pneumatisch über ein im Kugellager 21 angeordnetes Expansionselement 19, beispielsweise den in Fig. 7 dargestellten Schlauch 19.

Eine wie voranstehend beschriebene Haltevorrichtung 1 für medizinische Zwecke zeichnet sich dadurch aus, dass die zur exakten Positionierung des in der Instrumentenaufnahme 6 angeordneten medizinischen Instruments dienenden Gelenke 3 einfach zu handhaben und zu reinigen sind. Bei der Handhabung ist insbesondere ausschlaggebend, dass die Gelenke 3 hydraulisch oder pneumatisch lösbar sind, so dass der Operateur die Freigabe der Gelenke 3 auch mit nur einer Hand oder sogar freihändig bewerkstelligen kann.

### Bezugszeichenliste

- 1: Haltevorrichtung
- 2: Tragarm
- 2a: Tragarmteil
- 3: Gelenk
- 4: Einspannvorrichtung
- 5: Operationstisch
- 6: Instrumentenaufnahme
- 7: Gelenkteil
- 8: Gelenkteil
- 9: Federelement
- 10: Verzahnung
- 10a: Zahnkranzring
- 11: Spannelement
- 12: Rasthaken
- 13: zylindrischer Kern/Teilelement
- 14: äußerer Ring/Teilelement
- 15: Nut-Feder-Verbindung
- 16: Stift
- 17: Rastaufnahme
- 18: Ringscheibe
- 19: Expansionselement/Schlauch
- 20: Durchgangsöffnung
- 21: Kugellager
- 22: Halbkugel
- 23: Platte
- 24: Durchgangsbohrung
- 25: Federelement

## Patentansprüche

1. Haltevorrichtung für medizinische Zwecke mit einem Tragarm (2), an dessen distalem Ende mindestens ein medizinisches Instrument in einer Instrumentenaufnahme (6) festlegbar ist und mit mindestens einem Gelenk (3) zur Positionierung des Tragarms (2) und/oder des medizinischen Instruments, wobei das mindestens eine Gelenk (3) zwischen einer das Gelenk (3) freigebenden und einer das Gelenk (3) arretierenden Stellung verstellbar ist, wobei das mindestens eine Gelenk (3) aus mindestens zwei Gelenkteilen (7, 8) besteht, die über ineinandergreifende Rastelemente in Wirkverbindung miteinander treten, die im Wesentlichen senkrecht zur Längsrichtung des Tragarms (2) ausgerichtet sind, und wobei im Gelenk (3) mindestens ein hydraulisch oder pneumatisch aufweitbares Expansionselement (19) angeordnet ist, über das das mindestens eine Gelenk (3) hydraulisch oder pneumatisch in die das Gelenk (3) freigebende Stellung überführbar ist,
**dadurch gekennzeichnet,**
**dass** die ineinandergreifende Rastelemente an den Gelenkteilen (7, 8) angeordnet sind und die Gelenkteile (7, 8) über mindestens ein Federelement (9) gegeneinander vorgespannt sind und, dass das im Gelenk (3) angeordnete Expansionselement (19) als Schlauch (19) ausgebildet ist.

2. Haltevorrichtung nach Anspruch 1, **dadurch gekennzeichnet, dass** in mindestens einem der miteinander in Wirkverbindung bringbaren Gelenkteile (7 oder 8) eine Durchgangsöffnung (20) zum Durchführen des Schlauches (19) ausgebildet ist.

3. Haltevorrichtung nach Anspruch 1 oder 2, **dadurch gekennzeichnet, dass** der Tragarm (2) mehrteilig ausgebildet ist, wobei die einzelnen Tragarmteile (2a) über die Gelenke (3) verschwenkbar miteinander verbunden sind.

4. Haltevorrichtung nach einem der Ansprüche 1 bis 3, **dadurch gekennzeichnet, dass** die Rastelemente als an den einander zugewandten Stirnflächen der Gelenkteile (7, 8) ausgebildete Verzahnungen (10) ausgebildet sind.

5. Haltevorrichtung nach Anspruch 4, **dadurch gekennzeichnet, dass** zumindest ein Gelenkteil (7, 8) mehrteilig so ausgebildet ist, dass diese Teilelemente (13, 14) koaxiale Zahnkranzringe (10a) aufweisen.

6. Haltevorrichtung nach Anspruch 5, **dadurch gekennzeichnet, dass** die Zahnkranzringe (10a) der Teilelemente (13, 14) in der das Gelenk (3) freigebenden Stellung unabhängig voneinander relativ zueinander verdrehbar sind und in der das Gelenk (3) arretierenden Stellung verdrehfest gegeneinander blockiert sind.

7. Haltevorrichtung nach Anspruch 5 oder 6, **dadurch gekennzeichnet, dass** die einzelnen Teilelemente (13, 14) des mehrteiligen Gelenkteils (7) über Nut-Feder-Verbindungen (15) führend miteinander verbunden sind.

8. Haltevorrichtung nach einem der Ansprüche 5 bis 7, **dadurch gekennzeichnet, dass** ein Gelenkteil (7) zweiteilig aufgebaut ist und aus einem inneren zylindrischen Kern (13) und einem den Kern (13) koaxial umgebenden äußeren Ring (14) besteht.

9. Haltevorrichtung nach Anspruch 8, **dadurch gekennzeichnet, dass** beide über das Gelenk (3) miteinander verbundenen Bauteile, insbesondere Tragarmteile (2a), so an dem zweiteiligen Gelenkteil (7) festgelegt sind, dass ein Bauteil/Tragarmteil (2a) mit dem inneren zylindrischen Kern (13) verbunden ist und das andere Bauteil/Tragarmteil (2a) mit dem äußeren Ring (14) verbunden ist.

10. Haltevorrichtung nach einem der Ansprüche 5 bis 9, **dadurch gekennzeichnet, dass** die Verzahnung (10) des mit dem mehrteilig ausgebildeten Gelenkteil (7) in Wirkverbindung stehenden anderen Gelenkteils (8) so breit ausgebildet ist, dass diese in der das Gelenk (3) arretierenden Stellung mit den allen Zahnkranzringen (10a) des mehrteiligen Gelenkteils (7) in Eingriff steht.

11. Haltevorrichtung nach einem der Ansprüche 1 bis 3, **dadurch gekennzeichnet, dass** die Rastelemente als Stifte (16) und Rastaufnahmen (17) zur formschlüssigen Aufnahme der Stifte (16) ausgebildet sind.

12. Haltevorrichtung nach Anspruch 11, **dadurch gekennzeichnet, dass** die Rastaufnahmen (17) als gelochte Ringscheibe (18) an einem der Gelenkteile (7) ausgebildet sind, die mit den korrespondierenden Stiften (16) am anderen Gelenkteil (8) in Eingriff bringbar ist.

13. Haltevorrichtung nach einem der Ansprüche 1 bis 12, **dadurch gekennzeichnet, dass** sich das mindestens eine Federelement (9) zur Erzeugung der Vorspannung zwischen den Gelenkteilen (7, 8) an einem Gelenkteil (7 oder 8) abstützt und die Federkraft über ein mit dem Federelement (9) verbundenes Spannelement (11) auf das andere Gelenkteil (8 oder 7) überträgt.

14. Haltevorrichtung nach Anspruch 13, **dadurch gekennzeichnet, dass** das Spannelement (11) Rasthaken (12) zum Festlegen am anzudrückenden Gelenkteil (7) aufweist.

15. Haltevorrichtung nach einem der Ansprüche 1 bis 14, **dadurch gekennzeichnet, dass** die Vorspannkraft des Gelenks (3) über das mindestens eine Federelement (9) einstellbar ist.

16. Haltevorrichtung nach einem der Ansprüche 1 bis 15, **dadurch gekennzeichnet, dass** die Instrumentenaufnahme (6) als Kugellager (21) ausgebildet ist, wobei die Lagerkugel aus zwei Halbkugeln (22) besteht und die Lagerschale aus zwei gegeneinander verspannbaren und mit Ausnehmungen für die Lagerkugel versehenen Platten (23) besteht und wobei der Durchmesser der Ausnehmungen geringfügig kleiner als der Durchmesser der Lagerkugel.

17. Haltevorrichtung nach Anspruch 16, **dadurch gekennzeichnet, dass** im Bereich der Kontaktflächen der beiden die Lagerkugel bildenden Halbkugeln (22) eine Durchgangsbohrung (24) zur klemmenden Aufnahme des zu haltenden medizinischen Instruments ausgebildet ist.

18. Haltevorrichtung nach Anspruch 16 oder 17, **dadurch gekennzeichnet, dass** die die Lagerschale bildenden Platten (23) hydraulisch oder pneumatisch in eine die Lagerkugel freigebende Stellung überführbar sind.

19. Haltevorrichtung nach Anspruch 18, **dadurch gekennzeichnet, dass** im Kugellager (21) mindestens ein hydraulisch oder pneumatisch aufweitbares Expansionselement (19) angeordnet ist.

20. Haltevorrichtung nach Anspruch 19, **dadurch gekennzeichnet, dass** das Expansionselement (19) als Schlauch (19) ausgebildet ist.

## Claims

1. A holding device for medical purposes having a carrier arm (2), at the distal end of which at least one medical instrument can be secured in an instrument intake (6) and having at least one joint (3) for positioning the carrier arm (2) and/or the medical instrument, wherein the at least one joint (3) can be adjusted between a position that releases the joint (3) and a position that blocks the joint (3), wherein the at least one joint (3) is composed of at least two joint parts (7, 8) that enter into active engagement with one another via meshing latching elements, which are aligned essentially perpendicular to the longitudinal direction of the carrier arm (2), and wherein in the joint (3) at least one hydraulically or pneumatically expandable expansion element (19) is arranged, via which the at least one joint (3) can be converted hydraulically or pneumatically into the position releasing the joint (3), **characterized in that** the meshing latching elements are arranged on the joint parts (7, 8) and the joint parts (7, 8) are prestressed with respect to one another via at least one spring element (9), and that the expansion element (19) arranged in the joint (3) is embodied as a hose (19).

2. The holding device according to claim 1, **characterized in that** a through opening (20) for guiding through the hose (19) is embodied in at least one of the joint parts (7 or 8) that can be brought into active engagement with one another.

3. The holding device according to claim 1 or 2, **characterized in that** the carrier arm (2) is embodied in a multiple-part manner, wherein the individual carrier arm parts (2a) are connected to one another in a pivotable manner via the joints (3).

4. The holding device according to one of claims 1 through 3, **characterized in that** the latching elements are embodied as toothings (10) embodied on the front faces of the joint parts (7, 8) facing towards one another.

5. The holding device according to claim 4, **characterized in that** at least one joint part (7, 8) is embodied in a multiple-part manner such that these partial elements (13, 14) have coaxial wheel sprocket rings (10a).

6. The holding device according to claim 5, **characterized in that** the wheel sprocket rings (10a) of the partial elements (13, 14) in the position releasing the joint (3) can be rotated relative to one another independently of one another and in the position blocking the joint (3) are blocked against rotation with respect to one another.

7. The holding device according to claim 5 or 6, **characterized in that** the individual partial elements (13, 14) of the multiple-part joint part (7) are connected to one another in a guiding manner via tongue and groove connections (15).

8. The holding device according to one of claims 5 through 7, **characterized in that** one joint part (7) is structured in a two-part manner and is composed of an inner cylindrical core (13) and an outer ring (14) surrounding the core (13) coaxially.

9. The holding device according to claim 8, **characterized in that** both of the components connected to one another via the joint (3), in particular carrier arm parts (2a), are secured to the two-part joint part (7) such that one component/carrier arm part (2a) is connected to the inner cylindrical core (13) and the other component/carrier arm part (2a) is connected to the outer ring (14).

10. The holding device according to one of claims 5 through 9, **characterized in that** the toothing (10) of the other joint part (8) in active engagement with the joint part (7) embodied in a multiple-part manner is embodied so broadly that in the position blocking the joint (3) it is in engagement with all the wheel sprocket rings (10a) of the multiple-part joint part (7).

11. The holding device according to one of claims 1 through 3, **characterized in that** the latching elements are embodied as pins (16) and latching receptacles (17) for positive reception of the pins (16).

12. The holding device according to claim 11, **characterized in that** the latching receptacles (17) are embodied as perforated washer (18) on one of the joint parts (7), which can be brought into engagement with corresponding pins (16) on the other joint part (8).

13. The holding device according to one of claims 1 through 12, **characterized in that** the at least one spring element (9) for generating the prestressing between the joint parts (7, 8) is supported on one joint part (7 or 8) and transmits the spring force to the other joint part (8 or 7) via a clamping element (11) connected to the spring element (9).

14. The holding device according to claim 13, **characterized in that** the clamping element (11) has latching hooks (12) for securing on the joint part (7) to be pressed.

15. The holding device according to one of claims 1 through 14, **characterized in that** the prestressing force of the joint (3) can be adjusted via the at last one spring element (9).

16. The holding device according to one of claims 1 through 15, **characterized in that** the instrument intake (6) is embodied as a ball bearing (21), wherein the bearing ball is composed of two hemispheres (22) and the bearing bushing is composed of two plates (23) that can be braced with respect to one another and are provided with recesses for the bearing ball, and wherein the diameter of the recesses is slightly smaller than the diameter of the bearing ball.

17. The holding device according to claim 16, **characterized in that** in the region of the contact surfaces of the two hemispheres forming the bearing ball (22) a through bore (24) for the clamping accommodation of the medical instrument to be held is embodied.

18. The holding device according to claim 16 or 17, **characterized in that** the plates (23) forming the bearing bushing can be converted hydraulically or pneumatically into a position releasing the bearing ball.

19. The holding device according to claim 18, **characterized in that** at least one expansion element (19) that can be expanded hydraulically or pneumatically is arranged in the ball bearing (21).

20. The holding device according to claim 19, **characterized in that** the expansion element (19) is embodied as a hose (19).

## Revendications

1. Dispositif formant support de maintien destiné à des fins médicales, comprenant un bras porteur (2) à l'extrémité distale duquel peut être fixé au moins un instrument médical dans un logement de réception d'instrument (6), et comprenant au moins une articulation (3) pour le positionnement du bras porteur (2) et/ou de l'instrument médical, dispositif dans lequel ladite au moins une articulation (3) peut être réglée dans une position dans laquelle l'articulation (3) est libre ou dans une position dans laquelle l'articulation (3) est bloquée,
dans lequel ladite au moins une articulation (3) est constituée d'au moins deux composants d'articulation (7, 8) qui entrent en interaction réciproque par l'intermédiaire d'éléments d'encliquetage engrenant mutuellement et orientés sensiblement de manière perpendiculaire à la direction longitudinale du bras porteur (2), et
dans lequel dans l'articulation (3) est agencé au moins un élément d'expansion (19) expansible par voie hydraulique ou pneumatique, par l'intermédiaire duquel ladite au moins une articulation (3) peut être transférée, par voie hydraulique ou pneumatique, dans la position dans laquelle l'articulation (3) est libre, **caractérisé**
**en ce que** les éléments d'encliquetage engrenant mutuellement sont agencés sur les composants d'articulation (7, 8), et les composants d'articulation (7, 8) sont précontraints l'un vers l'autre par l'intermédiaire d'au moins un élément de ressort (9), et en ce que l'élément d'expansion (19) agencé dans l'articulation (3), est réalisé en tant que tuyau souple (19).

2. Dispositif formant support de maintien selon la revendication 1, **caractérisé en ce que** dans l'un au moins des composants d'articulation (7 ou 8) pouvant être amenés en interaction réciproque, est réalisé une ouverture de passage (20) pour y faire passer le tuyau souple (19).

3. Dispositif formant support de maintien selon la revendication 1 ou la revendication 2, **caractérisé en ce que** le bras porteur (2) est réalisé en plusieurs parties, les parties de bras porteur (2a) individuelles étant reliées les unes aux autres de manière pivotante, par l'intermédiaire des articulations (3).

4. Dispositif formant support de maintien selon l'une des revendications 1 à 3, **caractérisé en ce que** les éléments d'encliquetage sont réalisés en tant que dentures (10) formées sur les surfaces frontales dirigées l'une vers l'autre, des composants d'articulation (7, 8).

5. Dispositif formant support de maintien selon la revendication 4, **caractérisé en ce que** tout au moins un composant d'articulation (7, 8) est réalisé en plusieurs parties de façon à ce que ces parties ou éléments de composant (13, 14) présentent des anneaux de couronne dentée (10a) agencés coaxialement.

6. Dispositif formant support de maintien selon la revendication 5, **caractérisé en ce que** les anneaux de couronne dentée (10a) des parties de composant (13, 14) peuvent, dans la position libérant l'articulation (3), tourner relativement l'un par rapport à l'autre indépendamment l'un de l'autre, et sont bloqués en rotation l'un par rapport à l'autre, dans la position dans laquelle l'articulation (3) est bloquée.

7. Dispositif formant support de maintien selon la revendication 5 ou la revendication 6, **caractérisé en ce que** les parties de composant (13, 14) individuelles du composant d'articulation (7) en plusieurs parties, sont reliées mutuellement en étant guidées par l'intermédiaire de liaisons à rainure et languette (15).

8. Dispositif formant support de maintien selon l'une des revendications 5 à 7, **caractérisé en ce qu'**un composant d'articulation (7) est d'une construction en deux parties et est constitué d'un mandrin central cylindrique (13) intérieur, et d'un anneau extérieur (14) entourant coaxialement le mandrin central (13).

9. Dispositif formant support de maintien selon la revendication 8, **caractérisé en ce que** les deux pièces, notamment les parties de bras porteur (2a), mutuellement reliées par l'intermédiaire de l'articulation (3), sont fixées au composant d'articulation (7) en deux parties, de façon telle qu'une pièce/partie de bras porteur (2a) soit reliée au mandrin central cylindrique (13) intérieur, et l'autre pièce/partie de bras porteur (2a) soit reliée à l'anneau extérieur (14).

10. Dispositif formant support de maintien selon l'une des revendications 5 à 9, **caractérisé en ce que** la denture (10) de l'autre composant d'articulation (8), qui est en interaction avec le composant d'articulation (7) en plusieurs parties, est réalisée d'une largeur telle, que cette denture soit en prise avec tous les anneaux de couronne dentée (10a) du composant d'articulation (7) en plusieurs parties, dans la position dans laquelle l'articulation (3) est bloquée.

11. Dispositif formant support de maintien selon l'une des revendications 1 à 3, **caractérisé en ce que** les éléments d'encliquetage sont réalisés en tant que broches (16) et logements de réception d'encliquetage (17) destinés à loger, par complémentarité de formes, les broches (16).

12. Dispositif formant support de maintien selon la revendication 11, **caractérisé en ce que** les logements de réception d'encliquetage (17) sont réalisés sur l'un des composants d'articulation (7), sous la forme d'un disque annulaire perforé (18), qui peut être amené en prise avec les broches correspondantes (16) sur l'autre composant d'articulation (8).

13. Dispositif formant support de maintien selon l'une des revendications 1 à 12, **caractérisé en ce que** ledit au moins un élément de ressort (9) pour produire la précontrainte entre les composants d'articulation (7, 8), prend appui sur un composant d'articulation (7 ou 8), et transmet la force de ressort à l'autre composant d'articulation (8 ou 7) par l'intermédiaire d'un élément de serrage (11) relié à l'élément de ressort (9).

14. Dispositif formant support de maintien selon la revendication 13, **caractérisé en ce que** l'élément de serrage (11) présente des crochets d'encliquetage (12) pour la fixation au composant d'articulation (7) devant être pressé contre l'autre composant.

15. Dispositif formant support de maintien selon l'une des revendications 1 à 14, **caractérisé en ce que** la force de précontrainte de l'articulation (3) peut être réglée par l'intermédiaire dudit au moins un élément de ressort (9).

16. Dispositif formant support de maintien selon l'une des revendications 1 à 15, **caractérisé en ce que** le logement de réception d'instrument (6) est réalisé en tant que palier à rotule (21), la rotule de palier étant constituée de deux demi-rotules (22) et le coussinet de palier de deux plaques (23) qui peuvent être serrées l'une contre l'autre et sont pourvues d'évidements pour la rotule de palier, et le diamètre des évidements étant très légèrement inférieur au diamètre de la rotule de palier.

17. Dispositif formant support de maintien selon la revendication 16, **caractérisé en ce que** dans la zone des surfaces de contact des deux demi-rotules (22) formant la rotule de palier, est formée un alésage de passage (24) destiné à recevoir et loger par serrage l'instrument médical à maintenir.

18. Dispositif formant support de maintien selon la revendication 16 ou la revendication 17, **caractérisé en ce que** les plaques (23) formant le coussinet de palier peuvent être transférées, par voie hydraulique ou pneumatique, dans une position dans laquelle la rotule de palier est libre.

19. Dispositif formant support de maintien selon la revendication 18, **caractérisé en ce que** dans le palier à rotule (21) est agencé au moins un élément d'expansion (19) expansible par voie hydraulique ou pneumatique.

20. Dispositif formant support de maintien selon la revendication 19, **caractérisé en ce que** l'élément d'expansion (19) est réalisé en tant que tuyau souple (19).
